# EUROPEAN PATENT SPECIFICATION

(11) **EP 1 574 572 B1**
(45) Date of publication and mention of the grant of the patent: **23.05.2007**
(21) Application number: 04075764.3
(22) Date of filing: 09.03.2004
(51) Int. Cl.: C12N 15/11, C12N 9/22, C07H 21/00

(54) **Compounds for hydrolysing ribonucleic acids (RNAs)**
Verbindungen zur Hydrolyse von Ribonukleinsäuren (RNS)
Composés pour l'hydrolyse d'acides ribonucléiques (RNAs)

(43) Date of publication of application: 14.09.2005
(73) Proprietor: Prosensa B.V., 2333 CC Leiden (NL)
(72) Inventor: Wanner, Brigitte, 3705 TD Zeist (NL); Platenburg, Gerard, 2333 CC Leiden (NL)
(74) Representative: van Westenbrugge, Andries

(56) References cited:
- PATENT ABSTRACTS OF JAPAN vol. 1998, no. 12, 31 October 1998 (1998-10-31) -& JP 10 191970 A (KAGAKU GIJUTSU SHINKO JIGYODAN), 28 July 1998 (1998-07-28)
- KOMIYAMA, MAKOTO ET AL: "Ethylenediamine-oligo DNA hybrid as sequence-selective artificial ribonuclease" JOURNAL OF THE CHEMICAL SOCIETY, CHEMICAL COMMUNICATIONS , (1), 77-8 CODEN: JCCCAT; ISSN: 0022-4936, 1995, XP009037328
- USHIJIMA K ET AL: "Site-specific cleavage of tRNA by imidazole and/or primary amine groups bound at the 5'-end of oligodeoxyribonucleotides" BBA - GENERAL SUBJECTS, ELSEVIER SCIENCE PUBLISHERS, NL, vol. 1379, no. 2, 2 February 1998 (1998-02-02), pages 217-223, XP004276084 ISSN: 0304-4165
- VERHEIJEN ET AL.: "Efficient Hydrolysis of RNA by a PNA-Diethylenetriamine Adduct" ANGEW. CHEMIE. INT. ED., vol. 39, no. 2, 2000, pages 369-372, XP002298296

## Description

### FIELD OF THE INVENTION

The present invention relates to a method and compounds for hydrolysing nucleic acids. In particular it relates to compounds that can be used for the preferential cleavage of a phosphodiester bond at a specific position in RNA. The invention thus provides a useful tool for studies concerning molecular biological science, in the field of protein engineering and in the medical field, in particular in view of antisense strategies.

### BACKGROUND OF THE INVENTION

Antisense technology is based on the finding that DNA and/or RNA transcription or translation can be modulated using an oligonucleotide which binds to the target nucleic acid. Such antisense oligonucleotides are understood as nucleotides which are complementary to the actual DNA or RNA target nucleic acid and having a sequence oriented in opposite direction. When natural oligonucleotides are used in an antisense strategy, i.e. oligonucleotides with standard, natural bases and backbone, these should in general contain at least 17 bases to effectively activate RNaseH activity and thereby have a down-regulating effect on gene expression.

As the field developed, various modifications to the oligonucleotides have been proposed rendering them more stable under the conditions that they are used. In particular if antisense oligonucleotides are introduced in intact cells they are exposed to attack by RNA- and DNA-specific nucleases leading to a loss in activity. Modifications to inhibit degradation by nucleases that have been described are 2'-*O*-alkyl or alkenyl (allyl) or alkynyl nucleotides, locked nucleic acids (LNAs), peptide nucleic acids (PNAs), phosphorothioates, morpholino's, etc.

In another direction artificial ribonucleases have been attracting attention for their potential use for gene manipulation. In particular RNA-cleaving molecules have been bound to the terminal ends of oligonucleotides (DNAs). The oligonucleotides hybridise to a specific sequence in a target RNA followed by cleavage of the RNA at a specific site. In this respect in particular the susceptibility of the phosphodiester bond between a cytosine and adenosine (CA) to hydrolysis *via* intra-molecular acid-base cooperation has been exploited. Hydrolysis occurs at the 3'-side of the cytosine nucleotide.

Komiyama *et al.* reported in J. Chem. Soc., Chem. Commun., 1995, 77-78 that a hybrid of diethylenetriamine (DETA) anchored to the 5'-end of a DNA 19-mer by means of a carbamate bond hydrolysed linear RNA selectively at the 3'-end of cytosine to give a 22-mer RNA fragment with a 3'-phosphate terminus. The selective scission was ascribed to intra-molecular acid-base cooperation of an ammonium cation and a neutral amine in the ethylenediamine [-N(CH₂)₂NH₂] moiety of the DNA-DETA adduct. Only 10 mol% RNA was hydrolysed after incubation at pH 8 for 4 h at 50 °C.

Also JP 10 191970 discloses an artificial ribonuclease wherein the polyethylene diamine residue (= cutter) is bound to a midway site of the oligomer and is positioned directly adjacent to the position that is to be cleaved in the target RNA.

In an attempt to improve on this Verheijen described an adduct of DETA with a 10-mer PNA (Angew. Chem. Int. Ed. 2000, 39, 369 - 372). PNA was selected as oligonucleotide because it resists biodegradation and as PNAs bind more strongly to RNA (or DNA) than natural oligonucleotides an oligomer of 10 PNA monomers was of sufficient length for hybridisation. The DETA cleavage moiety was coupled to the PNA via a urea bond. In the target RNA an additional scission site was introduced by replacement of a guanosine for a cytosine. Indeed target RNA was hydrolysed at two positions and the total conversion for RNA hydrolysis was approximately 29% after incubation at pH 7 for 4 h at 40 °C (note that these conditions are a better representative of the physiological conditions than those employed by Komiyama).

The following is a schematic representation of these two prior art documents. First the complex of RNA (30-mer) with DNA-DETA as employed by Komiyama *et al.* is depicted followed by the complex of RNA (25-mer) with PNA-DETA from Verheijen. DETA stands for NH(CH₂)₂NH(CH₂)₂NH₂. Nucleotide units are written in upper case, PNA units in lower case. The arrows mark the cleavage position of RNA for hydrolysis by DNA-DETA or PNA-DETA. Note the guanosine in the Komiyama RNA is replaced at position 19 with a cytosine in the Verheijen RNA.

Komiyama

↓

[RNA]          5'- GGAGGUCCUGUGUUCGAUCCACAGAAUUCG - 3'

●●●●●●●●●●●●●●●●●●●

[DNA-DETA]     3'- TCCAGGACACAAGCTAGGT—O(C=O)—DETA

Verheijen:

6↓ 17↓19↓

[RNA] 5'- UCCUGUGUUCGAUCCACACAAUUCG - 3'

●●●●●●●●●●●

[PNA-DETA] 3'-caagctaggt—NH(C=O)-DETA

As already mentioned the Verheijen RNA is hydrolysed at two positions, *viz*. at the 3'-side of C17 and C19. After 7h of incubation, the ratio RNA:PNA-DETA was 1:33, approximately 50% of the RNA was hydrolysed: approximately 15% at the 3'-side of C17 and approximately 35% at the 3'-side of C19. In addition Verheijen noticed a minor degradation product resulting from scission at the 3'-side of U6. This scission is ascribed to the fact that a 10-mer PNA-oligomer corresponds to 1 helix-turn, which positions in an RNA•PNA-DETA complex the ethylenediamine residue in close proximity of the phosphodiester bond between U6 and G7.

Other relevant prior art in this field to consider is Komiyama et al.: "Molecular design of artificial hydrolytic nucleases and ribonucleases" in Nucleic Acids Symposium Series No. 29, 1993, 197-198. This document shows that from two possibly hydrolysable phosphodiester bonds in a target tRNA, the more distant bond three nucleotides away from the terminal hybridising nucleotide is more readily hydrolysed than the bond one nucleotide away from the terminal hybridising nucleotide. It should be noted however that the artificial hydrolysing nucleases from Komiyama *et al.* are the result of molecular modelling studies. This teaches the skilled reader that these nucleases are already the result of optimisation of the position of hybridisation and the position where hydrolysis takes place.

The nucleases from Komiyama have been the starting point for Verheijen et al. (see above) and for Vlassov *et al.,* see Antisense and Nucleic Acid Drug Development, 1997, vol. 7, 39-42. Both Verheijen *et al.* and Vlassov *et al.* arrive at the same conclusion as Komiyama that the more distant hydrolysable phosphodiester bond is more readily hydrolysed. Vlassov *et al.* suggest there is room for improvement of the hydrolysis or cleavage efficiency by optimising length and rigidity of the linker structure that links a polyamine-based cleaving group to an oligonucleotide and optimising the site of attachment of the linker to the oligonucleotide. It is not suggested to additionally vary the position of hybridisation of the oligonucleotide to a target RNA.

The present invention aims to improve on the state of the art compounds for hydrolysing nucleic acids by polyamine mediated cleavage by providing compounds with improved efficiency of hydrolysis thereby in particular increasing the rate of hydrolysis.

### SUMMARY OF THE INVENTION

It has been found that there is a relationship between the distance of a polyamine moiety that is responsible for hydrolysis of a nucleic acid to the oligonucleotide to which the polyamine is bonded and the position where the nucleic acid optimally is hydrolysed. Hereinafter the polyamine moiety that is responsible for hydrolysis is also called a Cutter. The oligonucleotide to which the Cutter is bonded is hereinafter also shortened to Oligo. The distance between the Cutter and the Oligo is established by a structure element covalently linking the two and hereinafter will be referred to as Spacer. Most conveniently the distance established by the Spacer is described in terms of number of atoms from Oligo to Cutter. The atoms that are counted are those forming a straight chain from Oligo to Cutter. Possible substituents or branching of such a chain do not contribute to the number of atoms that is defined by the Spacer.

Surprisingly the hydrolysis of a substrate RNA at the 3'-end of a cytosine in a cytosine-adenosine sequence was doubled compared to state of the art compounds if relative to the projected position that is to be hydrolysed complexation of the oligonucleotide ends at least 4 nucleotides away, and the distance between a polyamine moiety responsible for hydrolysis and the oligonucleotide is at least 2 atoms in a straight chain.

Thus the invention concerns a method for ribonucleic acid hydrolysis in which an Oligo or mimic thereof, which is conjugated to a Cutter comprising at least 2 nitrogen atoms, said 2 nitrogen atoms being involved in hydrolysis of said ribonucleic acid and there is a Spacer comprising at least 2 atoms in a straight chain linking a terminal nucleotide or mimic thereof of said Oligo and said Cutter, is hybridised at least 4 and at most 8 nucleotides away from the projected position that is to be hydrolysed.

In a further aspect the invention concerns a compound for ribonucleic acid hydrolysis said compound having a structure
Oligo-Spacer-Cutter
in which
Oligo is a ribonucleic acid of at least 8 nucleotides or mimic thereof, being capable of hybridising to a target nucleic acid in such a way that a terminal nucleotide or mimic thereof hybridises to a nucleotide at least 4 nucleotides and at most 8 nucleotides away from the position that is to be hydrolysed,
Spacer covalently links Cutter to said terminal nucleotide of said Oligo and comprises at least 2 atoms in a straight chain from Oligo to Cutter
Cutter is a polyamine having at least two nitrogen atoms.

### DETAILED DESCRIPTION OF THE INVENTION

In their search for methods to efficiently and reliably modulate gene expression, the present inventors have investigated the possibility of selectively hydrolysing target ribonucleic acids. In this respect attention was directed to artificial nucleases acting through polyamine mediated cleavage of phosphodiester bonds.

Although the prior art system reported by Verheijen (*vide supra*) after 7 h incubation hydrolysed an appreciable amount of approximately 50% target nucleic acid, after 24 h incubation only 85% target nucleic acid was hydrolysed. In addition it is noticed that the target nucleic acid contained two projected sites for hydrolysis and furthermore a third more accidental hydrolysis site was present. Thus the efficiency of hydrolysis of this prior art artificial nuclease leaves a lot to be desired, in particular with respect to target nucleic acids having not three or two but only one optional site for hydrolysis.

Based on the prior art target nucleic acids mentioned above the following synthetic RNA sequence was used:

1 5 10 15 20 25
5'- UCCUGUGUUCGAUCCGCAGAAUUCG - 3'

The projected position for hydrolysis is underlined. Hydrolysis occurs at the 3'-end of the underlined cytosine. When counting the number of nucleotides away from the position that is to be hydrolysed either the cytosine or adenosine is included, depending in which direction, either towards the 5'-end or 3'-end respectively, an Oligo hybridises. Thus, in the example of the RNA sequence given above, an Oligo of which a terminal nucleotide or mimic thereof hybridises at least 4 nucleotides away from the position that is to be hydrolysed, this terminal nucleotide hybridises to the first uracil (U13) in the 5'-end direction. In the 3'-end direction the position 4 nucleotides away from the position that is to be hydrolysed is the uracil in position 22 (U22).

For hydrolysis of the projected position in the target nucleic acid given above ethylene diamine was used as a Cutter. For varying Spacer length glycine residues were inserted between Oligo and Cutter.

Oligo's that hybridised two or three positions away from the position that is to be hydrolysed showed similar rates of hydrolysis, irrespective of the presence of no, one or two glycine residues in the Spacer. Incubation at pH 7 for 7 h at 40 °C, with a ratio RNA:Oligo-Spacer-Cutter of 1:33 gave modest, 10-20%, hydrolysis. After 24 h incubation hydrolysis varied between 25-30%.

However, an Oligo that hybridised four positions away from the position that is to be hydrolysed showed remarkable improvement of the amount target ribonucleic acid that was hydrolysed. In particular for a compound in which the spacer comprised 5 atoms the hydrolysis after incubation at pH 7 for 7 h at 40 °C, with a ratio RNA:Oligo-Spacer-Cutter of 1:33 was more than 50% and after 24 h more than 95%.

The term "Oligo" used herein refers to a ribonucleic acid or a mimic thereof. This includes derivatives of ribonucleic acids to render the Oligo more stable under physiological conditions. Examples of suitable derivatives are 2'-*O*-alkyl, alkenyl (allyl) or alkynyl nucleotides, 2'-deoxy nucleotides (DNA), and/or 2'-deoxy-2'-fluoro nucleotides. 2'-*O*-methyl and 2'-O-allyl nucleotides are preferred. Optionally also or alternatively the phosphodiester bond may derivatised, for example as an *O*-alkylated phosphodiester or preferably a phosphorothioate. An Oligo comprising mimics of nucleotides refers to a nucleotide comprising locked nucleic acids (LNAs), peptide nucleic acids (PNAs), morpholino's, etc.

Suitable bases in the Oligo include adenine, guanine, cytosine, uracil, thymine, inosine, 2,6-diaminopurine, xanthine, hypoxanthine, and further derivatives of such bases such as alkyl, amino, aza and/or halo substituted bases or deaza bases. Further modifications of the bases may be suitable as well and are known by those skilled in the art.

The Oligo according to this invention has a length of at least 8 nucleotides or mimic thereof. Usually for sufficient hybridisation a length of more than 25 nucleotides (or mimics) is not necessary. A length of at least 10 nucleotides (or mimic) is preferred.

An Oligo has two positions where possibly the Spacer can be attached. The Spacer is attached to either the 5'-*O* or the 3'-*O* atom. In case the Oligo comprises a mimic of a nucleotide at either end, the Spacer is attached to the atom in the mimic of the nucleotide that corresponds to the 5'-*O* or 3'-*O* atom.

The term "Spacer" is used herein to define a distance between Oligo and Cutter in terms of number of atoms. The number of atoms is counted in a straight chain from the first atom that is bonded to the 5'-*O* or 3'-*O* atom of a terminal nucleotide or mimic thereof in the Oligo to the last atom to which the Cutter is coupled. The Spacer ends at the first nitrogen atom in the Cutter that is involved in cleavage of a phosphodiester bond in a target RNA. The atoms in the spacer may be any suitable atom known to the skilled person, preferably the atoms in the Spacer, which may be the same or different, are selected from C, O, S, N and P.

The Spacer may be branched and/or the atoms in the Spacer may be substituted with groups having a functionality. Such substituents may have a functionality to enable detection of the compound of the invention, for instance the Spacer may be substituted with a fluorescent label. Such labels are widely known. Alternatively or additionally the Spacer may be substituted with a group that facilitates passage of the cell membrane. Such groups may be hydrophobic groups that readily interact with the lipid bilayer of a cell membrane and/or may be groups that interact with receptors or enzymes involved in transport across cell membranes.

It should be understood that the Spacer-Cutter moiety for instance entirely may consist of polyetheleneamine. As the first two atoms that are attached to the terminal nucleotide or mimic thereof in an Oligo are considered not to be involved in phosphodiester bond hysdrolysis and therefore are no part of the Cutter.

The term "Cutter" as used herein refers to a polyamine having at least two nitrogen atoms. The Cutter comprises at least one secondary nitrogen atom or tertiary nitrogen atom, optionally the Cutter also comprises at least one further secondary or tertiary nitrogen atom, preferably the Cutter comprises at least one further primary nitrogen atom. Polyamine mediated scission of phosphodiester bonds is a well known phenomenon in the art and the skilled person will be able to apply suitable Cutter moieties. Examples of suitable Cutters are ethylenediamine, diethylenetriamine, tris(2-aminoethyl)amine, ethyleneamine based dendrimers, pentaerythrityl tetraamine, spermine, spermidine, diaza-, triaza-, or tetraazacycloalkyl compounds, such as for instance piperazine, cyclen, tetraazacyclopentadecane, tetraazacyclotetradecane, tetraazaundecane, triazacyclononane, triazacyclododecane etc. and derivatives thereof.

In one embodiment the Oligo in the compound of the invention comprises PNA as nucleotide mimics.

In another embodiment the Oligo in the compound of the invention 2'-*O*-alkyl, preferably methyl or allyl, phosphorothioate as nucleotide mimics.

In one embodiment the compound according to the invention is one wherein said terminal nucleotide or mimic thereof of said Oligo hybridises 4 positions away from the position that is to be hydrolysed and the number of atoms in a straight chain from Oligo to Cutter is 4-6, preferably 5.

When the Oligo hybridises further than 4 positions away from the projected position to be hydrolysed of a ribonucleic acid it is preferred the Spacer increases in length. It one embodiment for each position more than 4 that the Oligo hydrolyses away from the projected position to be hydrolysed the Spacer is increased by at least 6 atoms in length. Thus for an Oligo-Spacer―Cutter that hybridises 5 atoms away from the projected position to be hydrolysed the Spacer comprises preferably at least 8 atoms, and for an Oligo-Spacer-Cutter that hybridises 6 atoms away from the projected position to be hydrolysed the Spacer comprises preferably at least 14 atoms, for an Oligo-Spacer-Cutter that hybridises 7 atoms away from the projected position to be hydrolysed the Spacer comprises preferably at least 20 atoms, and for an Oligo-Spacer-Cutter that hybridises 8 atoms away from the projected position to be hydrolysed the Spacer comprises preferably at least 26 atoms, etc.

In a further embodiment the compound according to the invention is one wherein said terminal nucleotide or mimic thereof of said Oligo hybridises 5 positions away from the position that is to be hydrolysed and the number of atoms in a straight chain from Oligo to Cutter is 10-12, preferably 11.

In yet a further embodiment the compound according to the invention is one wherein said terminal nucleotide or mimic thereof of said Oligo hybridises 6 positions away from the position that is to be hydrolysed and the number of atoms in a straight chain from Oligo to Cutter is 16-18, preferably 17.

In a further embodiment the compound according to the invention is one wherein said terminal nucleotide or mimic thereof of said Oligo hybridises 7 positions away from the position that is to be hydrolysed and the number of atoms in a straight chain from Oligo to Cutter is 22-24, preferably 23.

In yet a further embodiment the compound according to the invention is one wherein said terminal nucleotide or mimic thereof of said Oligo hybridises 6 positions away from the position that is to be hydrolysed and the number of atoms in a straight chain from Oligo to Cutter is 28-30, preferably 29.

The upper limit of the position of hybridisation of a terminal nucleotide or mimic thereof away from the projected position to be hydrolysed is 8, preferably 7, more preferably 6. The maximum length of the Spacer is 30 atoms.

The compounds of this invention can be synthesised by methods known in the art. Preferably the compounds are synthesised using automated procedures. For example an Oligo of desired length and composition can be routinely prepared in a DNA/RNA synthesiser. It is routine work for the skilled person to prepare Spacer-Cutter part comprising a Spacer of a desired length to which a desired Cutter is coupled. For an example see for instance Verheijen *et al.* (Angew. Chem. Int. Ed. 2000, 39, 369 - 372). Such a Spacer -Cutter part has a suitable functionality to couple to a 3'-*O* or 5'-*O* (preferred) of a terminal nucleotide or nucleoside. Alternatively it is common practice for the skilled person to replace a 5'-*O* by a 5'-*N* and attach a Spacer -Cutter part having suitable functionality to couple to a nitrogen, for instance according to the method described by Komiyama which is discussed above. See for suitable methods J. Org. Chem. 2000, 65, 4900 - 4907, Angew. Chem. Int. Ed., 2001, 40, 2004 - 2021 and more specific J.Org. Chem., 2003, 68, 609 - 612, Bioconjug. Chem., 2003, 14, 276-281. Alternatively disulfide bridge scan be used which requires the introduction of an 5'-*S*, or via conventional maleimide-coupling. Alternatively a suitable method makes use of standard phosphoramidite chemistry to couple the Spacer-Cutter part to a nucleoside. This allows the incorporation of any type of ribonucleotide (analogue) or mimic thereof in the compound of the invention, see for instance Bioconjugate Chem. 2002,13(5), 1071.

A suitable method to prepare a compound of the invention comprising PNA as nucleotide building blocks is described by Verheijen which is discussed above.

The present invention provides useful compounds and methods for a variety of therapeutic, diagnostic, agricultural, target validation, genomic discovery, genetic engineering and pharmacogenomic applications.

In one aspect the invention concerns a method for hydrolysing ribonucleic acid at a predetermined position in which a target ribonucleic acid is contacted with a compound according to the invention.

The compounds of the invention can be designed to inhibit gene expression through targeting of a variety of RNA molecules. In one embodiment, the compounds of the invention are used to target various RNAs corresponding to a target gene. Non-limiting examples of such RNAs include messenger RNA (mRNA), alternate RNA splice variants of target gene(s), post-transcriptionally modified RNA of target gene(s), pre-mRNA of target gene(s). If alternate splicing produces a family of transcipts that are distinguished by usage of appropriate exons, the instant invention can be used to inhibit gene expression through the appropriate exons to specifically inhibit or to distinguish among the functions of gene family members.

The compounds of the invention can be utilised as diagnostics, therapeutics and as research reagents and kits. They can be utilised in pharmaceutical compositions by adding an effective amount of a compound of the invention to a suitable pharmaceutically acceptable diluent or carrier. They further can be used for treating organisms having a disease characterised by the undesired production of a protein. The organism can be contacted with a compound of the invention having a sequence that is capable of specifically hybridising with a strand of target nucleic acid that codes for the undesirable protein. Thus to modulate gene expression it is preferred that the RNA portion which is to be modulated be preselected to comprise that portion of RNA which codes for the protein whose formation is to be modulated. Therefore, the Oligo to be employed is designed to be specifically hybridisable to the preselected portion of target RNA. In one embodiment the Oligo is one which is designed to specifically bind with mRNA which codes for the protein whose production is to be modulated.

The formulation of therapeutic compositions and their subsequent administration is believed to be within the skill of those in the art. In general, for therapeutics, a patient in need of such therapy is administered a compound in accordance with the invention, commonly in a pharmaceutically acceptable carrier, in doses ranging from 0.01 µg to 100 g per kg of body weight depending on the age of the patient and the severity of the disease state being treated. Further, the treatment may be a single dose or may be a regimen that may last for a period of time which will vary depending upon the nature of the particular disease, its severity and the overall condition of the patient, and is to be determined by a medical practitioner. In some cases it may be more effective to treat a patient with a compound of the invention in conjunction with other traditional therapeutic modalities.

The pharmaceutical compositions of the present invention may be administered in a number of ways depending upon whether local or systemic treatment is desired and upon the area to be treated. Administration may be topical (including ophthalmic, vaginal, rectal, intranasal, transdermal), oral or parenteral. Parenteral administration includes intravenous drip, subcutaneous, intraperitoneal or intramuscular injection, or intrathecal or intraventricular administration.

Formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful.

Compositions for oral administration include powders or granules, suspensions or solutions in water or non-aqueous media, capsules, sachets or tablets. Thickeners, flavoring agents, diluents, emulsifiers, dispersing aids or binders may be desirable.

Compositions for intrathecal or intraventricular administration may include sterile aqueous solutions which may also contain buffers, diluents and other suitable additives.

Formulations for parenteral administration may include sterile aqueous solutions which may also contain buffers, diluents and other suitable additives.

The present invention can be practiced in a variety of organisms ranging from unicellular prokaryotic and eukaryotic organisms to multicellular eukaryotic organisms. Any organism that utilises DNA-RNA transcription or RNA-protein translation as a fundamental part of its hereditary, metabolic or cellular machinery is susceptible to such therapeutic and/or prophylactic treatment. Seemingly diverse organisms such as bacteria, yeast, protozoa, algae, plant and higher animal forms, including warm-blooded animals, can be treated in this manner. Further, since each of the cells of multicellular eukaryotes also includes both DNA-RNA transcription and RNA-protein translation as an integral part of their cellular activity, such therapeutics and/or diagnostics can also be practiced on such cellular populations. Furthermore, many of the organelles, e.g. mitochondria and chloroplasts, of eukaryotic cells also include transcription and translation mechanisms. As such, single cells, cellular populations or organelles also can be included within the definition of organisms that are capable of being treated with the therapeutic or diagnostic compounds of the invention. As used herein, therapeutics is meant to include both the eradication of a disease state, killing of an organism, e.g. bacterial, protozoan or other infection, or control of aberrant or undesirable cellular growth or expression.

### EXAMPLES

### EXAMPLE 1

As suitable building block to be used in an automated standard DNA/RNA synthesiser the phosphoramidite depicted below is used. In the structure on the left below Linker is is part of the Spacer as described above and therefore may also be omitted from the general structure presented below. PG stands for a protecting group, preferably base-labile. Linker may be a glycine moiety and PG may be Fmoc as presented in the structure below. This phosphoramidite can be incorporated into any type of RNA (Oligo) or mimic thereof.

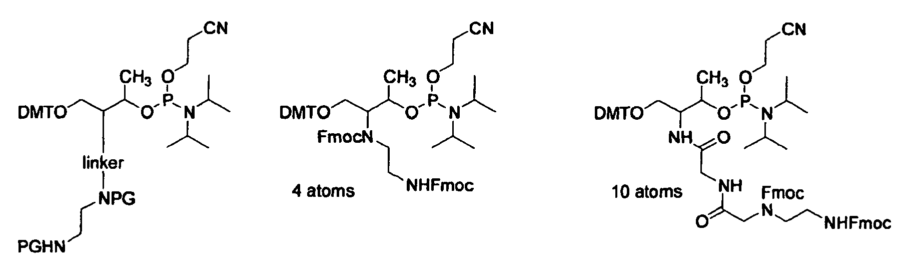

After incorporation of the phosphoramidite presented above in which Spacer has 10 atoms incorporation in an Oligo the structure represented below on the left is obtained. In this example the Spacer has 10 atoms.

By way of illustration Oligo mimics 2'-*O*-methyl phosphorothioate RNA and PNA are represented in the middle and on the right respectively.

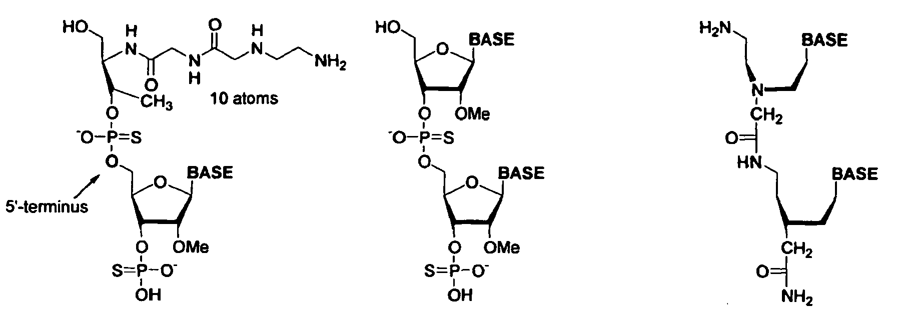

As an alternative a Spacer-Cutter moiety that can be linked to the 5'-terminus is used, see below. The Spacer length in this Spacer-Cutter moiety is variable. In the below detailed example the spacer is 4 and 10 atoms. It will be clear Spacer length can easily be varied.

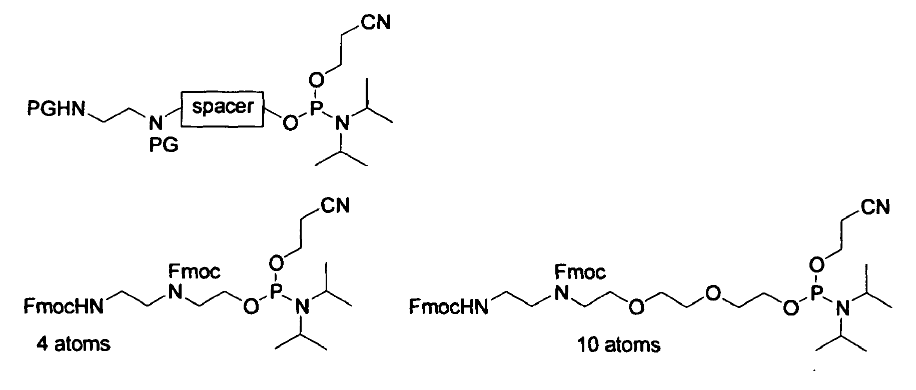

### EXAMPLE 2

Synthetic RNA (part of the tRNA^{Phe} having a point mutation: C-16 is replaced by G-16) used for cleavage experiments (underlined: the cleavage place):
5' - UCC UGU GUU CGA UCC GCA GAA UUC G - 3'

The following PNA-cutter sequences were synthesised, wherein Gly represents glycine, n = 0 - 2 and the Cutter is:

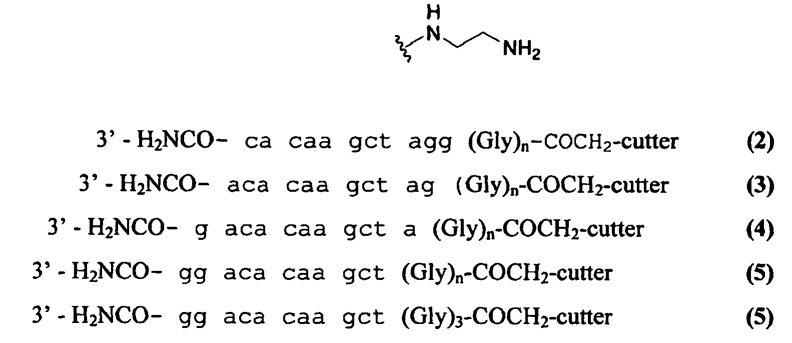

The number **(2), (3), (4)** and **(5)** refer to the number of positions that the Oligo hybridises away from the projected position to be hydrolysed. Each of **(2), (3), (4)** and **(5)** were varied in spacer length incorporating 0, 1 or 2 Gly residues.

### PNA synthesis

The PNAs used in this study were made by solid phase synthesis using a PNA synthesiser (Perseptive Biosystem, Expedite™ Nucleic Acid Synthesis System). All solvents (Biosolve) were used as received. The solid phase syntheses were performed on PEG-PS beads as solid support with rink-amide as linker (loading: 0.17 µmol·mg⁻¹). Assembly of the PNAs was established using the standard protocol described in the synthesiser manual, on a 2 µmol scale using Fmoc-chemistry and monomers in which the exocyclic amines were Bhoc-protected (Perseptive Biosystems). First couplings were double. Fmoc-Glycine and the diBoc-protected cutter building block were coupled to the *N*-terminus (5') of the PNA-oligomer while still on the resin, using the available positions in the synthesiser and the standard protocol for a double coupling. The diBoc-protected cutter building block (HO₂CCH₂NBocCH₂CH₂NHBoc) was synthesised by straightforward solution phase organic chemistry and fully characterised by its mass spectrum and ¹H- and ¹³C NMR-spectra.

The oligomer was cleaved from the resin and deprotected completely under acidic conditions (TFA /TIS/ H₂O, 9/ 1/ 1, v/ v/ v). RP-HPLC purification and analysis were carried out on a JASCO HPLC system equipped with an Altima C18 column (10 x 250 mm). Gradient elution was performed at 40 °C by building up a gradient starting with buffer A (0.1% TFA in water) and applying buffer B (0.1% TFA in acetonitrile/ water, 3/ 1, v/ v) with a flow rate of 4 mL/ min. The PNAs obtained were lyophilised and characterised by matrix-assisted laser desorption/ ionisation time-of-flight mass spectrometry (MALDI-TOF MS) and RP-HPLC.

### Cleavage experiments:

³²P 5'-Labeled RNA 25-mer was treated with PNA-cutter in TRIS-buffer (10 mM, pH 7) containing NaCl (100 mM) and EDTA (0.1 mM) to give the following concentrations: [RNA] = 60 nM, [PNA-cutter] = 2µM. The samples (70 µL) were incubated at 40 °C during 7 or 24 h. After this time the RNA was precipitated immediately by addition of NaOAc (3 M, pH 5, 7 µL), EtOH (225 µL) and 10 µg/ µL tRNA (1 µL). The precipitated RNA was recovered by centrifugation, dried and redissolved in water (5 µL) and loading buffer (5 µL). The solutions were heated at 90 °C for 1 minute, centrifuged and analysed on a denaturing 20% polyacrylamide electrophoresis gel containing 8 M urea. The controls used were: RNA, RNA base ladder and T1 digestion of the RNA.

### Results

Control RNA was hydrolysed for approximately 10% after 7 and after 24 hours of incubation. For compounds series **(2)** and **(3)** RNA was hydrolysed for approximately 20% or less after 7 hours of incubation and for approximately 40% or less after 24 hours of incubation.
Only from compounds series **(4)** RNA was hydrolysed at a reasonable rate, *viz*. for N = 0, 1 and 2 the RNA was hydrolysed for at least approximately 30% after 7 hours of incubation. Near quantitative hydrolysis was obtained for compound (4), n = 1 after 24 hours of incubation.

Optimisation with compound (**4**), n = 1 revealed that after 2 hours of incubation already approximately 50% RNA was hydrolysed at ratio RNA/compound of the invention of 1:10. At a ratio of 1:40 after 2 hours already approximately 75% was hydrolysed. At ratios of 1:10 and 1:20 after respectively 8 hours and 6 hours 75% or more RNA was hydrolysed. For the latter ratio after 24 hours incubation near quantitative hydrolysis was obtained.

Optimal hydrolysis for the **(5)** series compounds starts for n = 2.

### EXAMPLE 3

Synthetic RNA (part of the *E. Coli AcpP* mRNA) used for cleavage experiments (underlined: the cleavage place):
5' - A UUU AAG AGU AUG AGC ACU AUC GAA- 3'

The following PNA-cutter sequences were synthesised, wherein Gly represents glycine, and the Cutter is: 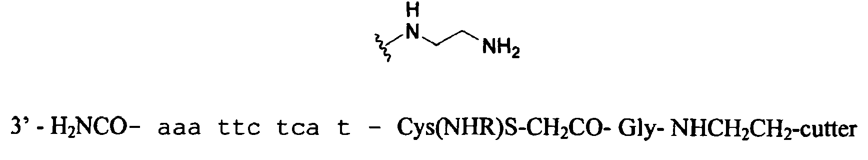 wherein R =

Ac or **(A)**

CH₂NHCO(CH₂)₄NH-Lys(PhePheLys)₃-NH₂ or **(B)**

CH₂(OCH₂CH₂)₂NH-Lys(PhePheLys)₃-NH₂ **(C)**

Note that compounds B and C comprise the peptide Lys(PhePheLys)₃ in the spacer to assist the compound passing the cell membrane.

### In vitro RNA degradation studies

All buffers were made from highly purified Milli Q water and were sterilised before use. The radioactively labeled RNA 25-mer and the conjugate to be tested (A, B, C, as controls RNA ans C without Cutter) were mixed in a Tris.HCl buffer (10mM, pH 7) containing NaCl (100mM) and EDTA (0.1mM) to give the following concentrations: [RNA] = 60nM, [Conjugate] = 2µM, as determined by A₂₆₀ units. The samples (70µL) were incubated at 40 °C for 16h. After incubation, the RNA was immediately precipitated by addition of 3M NaOAc (pH 5, 7µL), EtOH (225µL) and 10µg µL⁻¹ tRNA (1µL). The precipitated RNA was recovered by centrifugation, dried and then redissolved in 5µL H₂O and 5µL loading buffer. The solutions were heated at 80 °C for 1 min, centrifuged and analysed on a 20% denaturing electrophoresis gel. The gel was exposed to a phosphor screen (Molecular Dynamics) and the intensity of the RNA fragments was quantified by scanning the exposed screen on the Personal Molecular Imager FX System (Bio-Rad) followed by computer analysis with Quantity One software (Bio-Rad).

### Results

Compounds A, B and C and negative controls were incubated with the 25-mer ³²P-labeled RNA having a sequence resembling part of the *E.Coli AcpP* mRNA. After 16h at 40°C and pH 7, the RNA fragments were precipitated, applied to a 20% denaturing electrophoresis gel and quantified.
Gratifyingly, incubation of the RNA with the conjugates **B** and **C** as well as the peptide-lacking PNA-DETA **A,** led to the formation of two major RNA degradation products stemming from cleavage at the marked CA and UA sites. The cleavage sites were determined by comparison of the mobility of the bands with those of a T1 digest of the RNA and a basic hydrolysis ladder. The intensity of the fragment spots revealed that incubation of the RNA with PNA-DETA **A** resulted in significant cleavage (20%) of the RNA compared to the negative controls (*i.e.* PNA-peptide conjugate without Cutter and RNA without any conjugate added), which only showed background cleavage of approximately 3.5%. An unexpected difference between the hydrolysis efficiency of conjugates **B** and **C** was observed. Conjugate **B** showed an even improved cleavage efficiency (30%) compared to the positive control **18** (20%), whereas conjugate C cleaved 10% of the target RNA.

### EXAMPLE 4

### Oligo comprising Spacer-Cutter at the 3'-terminus

Commercially available resin comprising Dde linker is used for PNA synthesis.

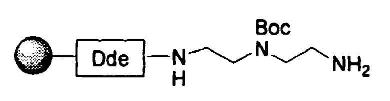

Starting from this resin a subsequent PNA synthesis results in the following Oligo:

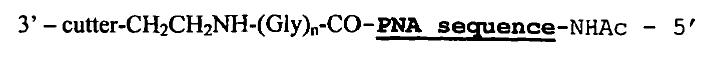

The Cutter herein is defined as :

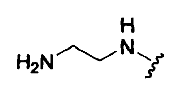

The "CO" is the 3'-terminus of the PNA-oligo and NHAc is the 5'-terminus.

For Spacer-Cutter at 3'-terminus of DNA/RNA based oligo's the strategy as outlined in Example 1 is applied

## Claims

1. A compound for nucleic acid hydrolysis said compound having a structure
Oligo-Spacer-Cutter
in which
Oligo is a ribonucleic acid of at least 8 nucleotides or mimic thereof, being capable of hybridising to a target nucleic acid in such a way that a terminal nucleotide or mimic thereof hybridises to a nucleotide at least 4 nucleotides and at most 8 away from the position that is to be hydrolysed,
Spacer covalently links Cutter to said terminal nucleotide of said Oligo and comprises at least 2 atoms in a straight chain from Oligo to Cutter
Cutter is a polyamine having at least two nitrogen atoms.

2. The compound according to claim 1 in which said Oligo comprises PNA as nucleotide mimics.

3. The compound according to claim 1 in which said Oligo comprises 2'-*O*-alkyl, preferably methyl, phosphorothioate as nucleotide mimics.

4. The compound according to any of preceding claims in which said Spacer comprises one or more amino acids, in particular glycine.

5. The compound according to any of preceding claims in which said Spacer is coupled to said Oligo *via* an amide bond.

6. The compound according to any of preceding claims in which said Cutter is ethylene diamine.

7. The compound according to any of preceding claims in which said terminal nucleotide or mimic thereof of said Oligo is 4 positions away from the position that is to be hydrolysed and the number of atoms in a straight chain from Oligo to Cutter is 4-6, preferably 5.

8. The compound according to any of preceding claims in said terminal nucleotide or mimic thereof of said Oligo is 5 positions away from the position that is to be hydrolysed and the number of atoms in a straight chain from Oligo to Cutter is 10-12, preferably 11.

9. A method for in vitro hydrolysing ribonucleic acid at a predetermined position in which a target ribonucleic acid is contacted with a compound according to any of the preceding claims.

10. The method according to claim 9 in which the ribonucleic acid is RNA, in particular mRNA.

11. Pharmaceutical composition comprising a compound according to any of claims 1-8 and a pharmaceutically acceptable carrier or diluent.

12. A compound according to any of claims 1-8 for use as a medicament.

13. A compound according to any of claims 1-8 for use as a diagnostic tool.

## Patentansprüche

1. Verbindung für eine Nukleinsäurehydrolyse, wobei die Verbindung eine Struktur
Oligo-Abstandshalter-Abschneider
aufweist, in der
Oligo eine Ribonukleinsäure mit mindestens 8 Nukleotiden oder ein Imitator dafür ist, der in der Lage ist, die Zielnukleinsäure auf eine solche Weise zu hybridisieren, dass ein endständiges Nukleotid oder ein Imitator dafür zu einem Nukleotid mit mindestens 4 Nukleotiden und höchstens 8 entfernt von der zu hydrolysierenden Position hybridisiert,
Abstandshalter den Abschneider kovalent mit dem endständigen Nukleotid von Oligo verbindet und mindestens zwei Atome in einer geraden Kette von Oligo zum Abschneider umfasst,
Abschneider ein Polyamin mit mindestens zwei Stickstoffatomen ist.

2. Verbindung nach Anspruch 1, bei der Oligo PNA als Nukleotidimitatoren umfasst.

3. Verbindung nach Anspruch 1, bei der Oligo 2'-*O*-Alkyl-, vorzugsweise -Methyl-, -phosphorthioat als Nukleotidimitatoren umfasst.

4. Verbindung nach einem der vorhergehenden Ansprüche, bei der der Abstandshalter ein oder mehrere Aminosäuren umfasst, insbesondere Glycin.

5. Verbindung nach einem der vorhergehenden Ansprüche, bei der der Abstandshalter über eine Amidbindung an Oligo gekoppelt ist.

6. Verbindung nach einem der vorhergehenden Ansprüche, bei der der Abschneider Ethylendiamin ist.

7. Verbindung nach einem der vorhergehenden Ansprüche, bei der das endständige Nukleotid oder der Imitator dafür von Oligo 4 Positionen entfernt von der zu hydrolysierenden Position ist und die Zahl der Atome in einer geraden Kette von Oligo zum Abschneider 4 bis 6, vorzugsweise 5 beträgt.

8. Verbindung nach einem der vorhergehenden Ansprüche, bei der das endständige Nukleotid oder der Imitator dafür von Oligo 5 Positionen entfernt von der zu hydrolysierenden Position ist und die Zahl der Atome in einer gerade Kette von Oligo zum Abschneider 10 bis 12, vorzugsweise 11 beträgt.

9. Verfahren zum in vitro-Hydrolysieren von Ribonukleinsäure an einer vorgegebenen Position, bei dem eine Zielribonukleinsäure mit einer Verbindung gemäß einem der vorhergehenden Ansprüche in Kontakt gebracht wird.

10. Verfahren nach Anspruch 9, bei dem die Ribonukleinsäure RNA, insbesondere mRNA ist.

11. Pharmazeutische Zusammensetzung, die eine Verbindung gemäß einem der Ansprüche 1 bis 8 und einen pharmazeutisch annehmbaren Träger oder ein pharmazeutisch annehmbares Verdünnungsmittel enthält.

12. Verbindung nach einem der Ansprüche 1 bis 8 zur Verwendung als Medikament.

13. Verbindung nach einem der Ansprüche 1 bis 8 zur Verwendung als diagnostisches Werkzeug.

## Revendications

1. Composé pour hydrolyser un acide nucléique, ledit composé ayant une structure
Oligo-Espaceur-Coupeur
dans lequel
L'oligo est un acide ribonucléique d'au moins 8 nucléotides, ou une imitation de celui-ci, qui est capable de s'hybrider à un acide nucléique cible de telle manière qu'un nucléotide terminal, ou une imitation de celui-ci, s'hybride à un nucléotide qui est éloigné d'au moins 4 et d'au plus 8 nucléotides par rapport à la position qui doit être hydrolysée,
L'espaceur se lie de manière covalente à un coupeur au niveau dudit nucléotide terminal dudit oligo et comprend au moins 2 atomes dans une chaîne linéaire à partir de l'oligo jusqu'au coupeur
Le coupeur est une polyamine ayant au moins deux atomes d'azote.

2. Composé selon la revendication 1, dans lequel ledit oligo comprend des PNA en tant qu'imitations de nucléotide.

3. Composé selon la revendication 1 dans lequel ledit oligo comprend 2'-*O*-alkyle, de préférence méthyle, phosphorothioate en tant qu'imitations de nucléotide.

4. Composé selon l'une quelconque des revendications précédentes, dans lequel ledit espaceur comprend un ou plusieurs acides aminés, en particulier la glycine.

5. Composé selon l'une quelconque des revendications précédentes, dans lequel ledit espaceur est couplé audit oligo via une liaison amide.

6. Composé selon l'une quelconque des revendications précédentes, dans lequel ledit coupeur est l'éthylène diamine.

7. Composé selon l'une quelconque des revendications précédentes, dans lequel ledit nucléotide terminal dudit oligo, ou une imitation de celui-ci, est éloigné de 4 positions par rapport à la position qui doit être hydrolysée, et le nombre d'atomes dans une chaîne linéaire à partir de l'oligo jusqu'au coupeur est de 4-6, de préférence 5.

8. Composé selon l'une quelconque des revendications précédentes, dans lequel ledit nucléotide terminal dudit oligo, ou une imitation de celui-ci, est éloigné de 5 positions par rapport à la position qui doit être hydrolysée, et le nombre d'atomes dans une chaîne linéaire à partir de l'oligo jusqu'au coupeur est de 10-12, de préférence 11.

9. Procédé pour hydrolyser un acide ribonucléique in vitro en une position prédéterminée, dans lequel un acide ribonucléique cible est mis en contact avec un composé selon l'une quelconque des revendications précédentes.

10. Procédé selon la revendication 9, dans lequel l'acide ribonucléique est de l'ARN, en particulier de l'ARNm.

11. Composition pharmaceutique comprenant un composé selon l'une quelconque des revendications 1 à 8 et un véhicule ou un diluant pharmaceutiquement acceptable.

12. Composé selon l'une quelconque des revendications 1 à 8 destiné à une utilisation en tant que médicament.

13. Composé selon l'une quelconque des revendications 1 à 8 destiné à une utilisation en tant qu'outil de diagnostic.
